# EUROPEAN PATENT APPLICATION

(11) **EP 4 528 746 A1**
(43) Date of publication of application: **26.03.2025**
(21) Application number: 23199372.6
(22) Date of filing: 25.09.2023
(51) Int. Cl.: G16H 30/20, G16H 40/40

(54) **UPGRADING MEDICAL IMAGING SYSTEMS**

(71) Applicant: Koninklijke Philips N.V., 5656 AG Eindhoven (NL)
(72) Inventor: JOHNSON, Mark Thomas, 5656AG Eindhoven (NL); VOGTMEIER, Gereon, 5656AG Eindhoven (NL); LEUSSLER, Christoph Günther, 5656AG Eindhoven (NL)
(74) Representative: Philips Intellectual Property & Standards

(57) **Abstract**

Medical imaging devices are required to be upgraded as part of a sustainable product development and lifecycle design. Since medical imaging devices are complex, implementation of the upgrades presents challenges. There is therefore provided an upgrade component configured to be installed in a medical imaging system. The upgrade component comprises: system circuitry configured to upgrade system functionality of the medical imaging system; and maintenance circuitry configured to upgrade maintenance functionality of the medical imaging system. In this way, installation of the new upgrade component in the medical imaging system triggers not only an upgrade to the system functionality but also an upgrade to the maintenance functionality.

## Description

### FIELD OF THE INVENTION

The invention relates to systems and methods for upgrading medical imaging systems.

### BACKGROUND OF THE INVENTION

Medical imaging devices are required to be upgraded as part of a sustainable product development and lifecycle design. Upgrades typically involve software updates for hardware that is compatible with the upcoming software improvements, or in some cases also modifications to the hardware. In view of the complexity of medical imaging devices, implementation of the upgrades presents challenges.

### SUMMARY OF THE INVENTION

To better address one or more of these concerns, there is provided, in a first aspect of invention an upgrade component configured to be installed in a medical imaging system, wherein the upgrade component comprises:
system circuitry configured to upgrade system functionality of the medical imaging system; and
maintenance circuitry configured to upgrade maintenance functionality of the medical imaging system.

In this way, installation of the new upgrade component in the medical imaging system triggers not only an upgrade to the system functionality but also an upgrade to the maintenance functionality.

In particular, the maintenance circuitry is configured to upgrade the maintenance functionality of the medical imaging system by providing upgraded maintenance functionality (specially) adapted to the upgraded system functionality.

The maintenance circuitry is configured to upgrade the maintenance functionality of the medical imaging system by providing a hardware upgrade as well as a software upgrade to the maintenance functionality of the medical imaging system. The hardware upgrade may comprise at least one additional sensor and/or additional processing circuitry, for example. The software upgrade may comprise analytics software, for example.

The medical imaging system may be a magnetic resonance imaging (MRI) system, a positron emission tomography (PET) system, an image-guided hyperthermia (IGHT) system, a singlephoton emission computed tomography (SPECT) system, a computed tomography (CT) system, an ultrasound (US) imaging system, a digital fluoroscopy system, a digital X-ray (DXR) system, a tomographic medical imaging system, or a radiological medical imaging system. In other examples, the medical apparatus may comprise multiple imaging modalities. It will furthermore be appreciated that systems and methods disclosed herein are not limited to medical imaging systems but are equally applicable to non-medical systems.

The upgrade component may be any new component to be installed in the medical imaging system. In one example, the upgrade component is an imaging chain component. The imaging chain component may be a data measurement component used to measure or record scan data (e.g., k-space data). For example, the data measurement component may be a magnetic resonance imaging coil or an X-ray detector. The X-ray detector may be a computed tomography detector, a digital X-ray detector, or a flat-panel X-ray detector, for example. In other examples, the imaging chain component is an X-ray source, an X-ray tube, an X-ray tube power supply, a high-voltage generator, or a controllable collimator. The upgrade component may alternatively be a sensor module, a support module (such as a cooling module), or a temperature simulation tool, for example for an X-ray tube of the medical imaging (e.g. CT or DXR) system.

The upgrade component may be configured to execute, following its connection to the medical imaging system, an upgrade procedure wherein instructions are sent from the maintenance circuitry of the upgrade component to the medical imaging system. The instructions may instruct the medical imaging system in how to adapt its system functionality and/or maintenance functionality to accommodate the upgrade component. For example, the instructions may cause the medical imaging system to update its own maintenance circuitry with information on how to maintain the upgrade component. The instructions may cause the medical imaging system to update a log file to record information from the upgrade component. The instructions may indicate whether the maintenance functionality of the medical imaging system is to be adapted to the operation of the upgrade component. The instructions may indicate whether the upgrade component will increase demands on the operation of one or more components of the medical imaging system. Further examples of system functionality and/or maintenance functionality which may be adapted according to the instructions may comprise any one or more of: switching patterns; warm up procedures; shut down procedures; sequences of switching; parameter settings; data paths; transmission techniques including ways of reading and processing data; sampling techniques; temperature ranges; cooling.

The upgrade procedure may further comprise transmissions from the medical imaging system to the newly-installed upgrade component. For example, the upgrade procedure may comprise the medical imaging sending - and the upgrade component receiving - a confirmation that the instructions have been successfully actioned. The upgrade procedure comprise the medical imaging sending - and the upgrade component receiving - a conflict indication, wherein the conflict indication indicates that the maintenance circuitry of the upgrade component is to adapt its operation, for example by executing one or more tasks previously executed by the medical imaging system.

The upgrade procedure may further comprise performing checks with respect to one or more of: electromagnetic compatibility; hardware and/or software compatibility; calibration; optimization; and patient safety. These checks may be performed by the imaging system, by the new upgrade component, or by both working together in combination. In one non-limiting example, the upgrade component operates in different modes, for example at different temperatures, which are associated with new monitoring modes and temperature log files. A check may be performed to ascertain whether the new upgrade component affects the image acquisition of the medical imaging device, for example whether there is additional noise influencing the imaging process and as a consequence whether there would be a visible change in the images. Such an EMC check for influence on image quality may be triggered following installation of the new component for all potential operation modes.

In one example, the system circuitry is configured to upgrade the system functionality of the medical imaging system by upgrading an analogue data transmission path to a digital data transmission path, and wherein the maintenance circuitry is configured to upgrade analog signal quality monitoring circuitry of the medical imaging system to digital signal quality monitoring circuitry. For example, the system circuitry may be configured to upgrade the system functionality of the medical imaging system by upgrading an analogue data transmission path to a digital data transmission path. In this case, the maintenance circuitry may be configured to determine a digital data loss caused by the upgrade. The maintenance circuitry may be further configured to determine an appropriate usage of error correction codes (e.g., CRC check) based on the digital data loss. Prior to the upgrade, analog monitoring circuitry may check the noise level, the signal-to-noise ratio and the presence of disturbing signals using peak detection. After the upgrade, the maintenance functionality comprises adapted, digital monitoring circuitry. Errors may be visible in data loss, which become more visible in higher error rates measurable in the CRC. The maintenance functionality of the system is thus upgraded to ascertain and/or reduce losses in digital data transmission. In this regard, the log file, the processing of the log file and/or error messages may be upgraded based on the analysis of CRC rates.

The present disclosure further envisages a modular implementation, in which the maintenance circuitry of the upgrade component and the maintenance functionality of the medical imaging system are comprised in respective maintenance modules.

According to a second aspect, there is provided a medical imaging system comprising the upgrade component of any preceding claim.

According to a third aspect, there is provided a method of upgrading a medical imaging system, the method comprising installing an upgrade component in a medical imaging system, wherein the upgrade component comprises system circuitry configured to upgrade system functionality of the medical imaging system and maintenance circuitry configured to upgrade maintenance functionality of the medical imaging system.

According to a fourth aspect, there is provided a computing system configured to perform the upgrade procedure as described herein.

According to a fifth aspect, there is provided a computer program (product) comprising instructions which, when executed by a computing system, enable or cause the computing system to perform the upgrade procedure described herein.

According to a sixth aspect, there is provided a computer-readable (storage) medium comprising instructions which, when executed by a computing system, enable or cause the computing system to perform the upgrade procedure described herein. The computer-readable medium may be transitory or non-transitory, volatile or non-volatile.

The term "circuitry", as used herein, may refer to hardware, firmware, and/or software configured to perform any of the operations or algorithms described herein. Hardware may comprise, singly or in any combination, hardwired circuitry, programmable circuitry such as computer processors comprising one or more individual instruction processing cores, or state machine circuitry. Firmware may be embodied as code, instructions and/or data stored or hardcoded in memory devices (e.g., non-volatile memory devices). Software may be embodied as a software package, code, instructions and/or data recorded on at least one transitory or non-transitory computer readable storage medium.

The term "obtaining", as used herein, may comprise, for example, receiving from another system, device, or process; receiving via an interaction with a user; loading or retrieving from storage or memory; measuring or capturing using sensors or other data acquisition devices.

The term "determining", as used herein, encompasses a wide variety of actions, and may comprise, for example, calculating, computing, processing, deriving, investigating, looking up (e.g., looking up in a table, a database or another data structure), ascertaining, and the like. Also, "determining" may comprise receiving (e.g., receiving information), accessing (e.g., accessing data in a memory), and the like. Also, "determining" may comprise resolving, selecting, choosing, establishing and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

The above-described aspects will become apparent from, and elucidated with, reference to the detailed description provided hereinafter.

### BRIEF DESCRIPTION OF THE DRAWINGS

A detailed description will now be given, by way of example only, with reference to the accompanying drawings, in which:
FIG. 1 schematically illustrates a medical imaging system in which a new component is to be installed to upgrade system functionality and maintenance functionality of the medical imaging system;
FIG. 2 is a flowchart illustrating an upgrade procedure which is carried out following connection of the new component to the medical imaging system; and
FIG. 3 shows the medical imaging system of FIG. 1 in more detail.

### DETAILED DESCRIPTION OF EMBODIMENTS

The present disclosure proposes an upgrade component for installation into a medical imaging system that enables, besides new system functionality, also new maintenance functionality adapted to the new system functionality.

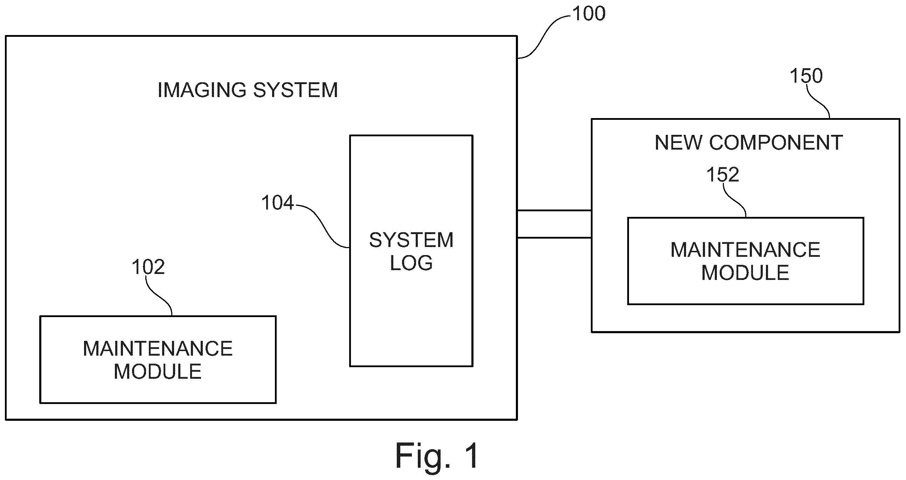

The medical imaging system 100 as shown in FIG. 1 implements system functionality for acquiring medical imaging data. The medical imaging system 100 comprises a maintenance module 102, which implements maintenance functionality for the medical imaging system 100, together with a system log file 104 for data logging of system relevant measurement data and system data. The maintenance module 102 comprises hardware components (such as sensors) and software components (such as analytics software) in order to assist in maintenance, testing, and/or servicing of the medical imaging system 100.

FIG. 1 further illustrates a new component 150 which is to be installed in the medical imaging system 100. The new component 150 comprises system circuitry configured to upgrade the system functionality of the medical imaging system 100. The new component 150 is further provided with its own maintenance module 152, which again may comprise both hardware and software components. The maintenance module 152 comprises maintenance circuitry configured to upgrade the maintenance functionality of the medical imaging system 100.

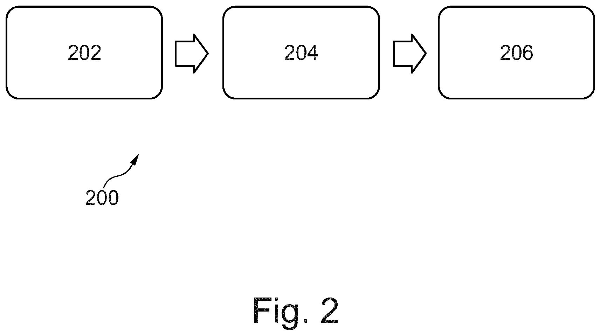

FIG. 2 is a flowchart illustrating an upgrade procedure 200 which is carried out following connection of the new component 150 to the existing medical imaging system 100.

In step 202, instructions are sent from the maintenance module 152 of the new component 150 to the medical imaging system 100. The instructions can cause the medical imaging system 100 to perform one or more of the following operations: update the maintenance module 102 with details of how to maintain and service the new component 150; update the log file 104 to record new information from the new component 150 (for example, related to new sensors associated therewith); indicate whether the existing maintenance functionality of the medical imaging system 100 needs to be adapted due to the operation of the new component 150, for example, whether operation of the new component is to put extra demands on the operation of other components of the medical imaging system 100.

In step 204, instructions are optionally sent from the medical imaging system 100 (e.g., from its maintenance module 102) to the new component 150 in order either: to confirm that the instructions received from the new component 150 have been successfully carried out; or to indicate where there are issues or conflicts necessitating the maintenance module 152 of the new component 150 to adapt its operation, for example by taking over one or more of the tasks originally previously executed by the medical imaging system 100.

In step 206, the upgrade procedure concludes with a validation step, in which the medical imaging system 100 cooperates with the newly-installed component 150 to perform one or more checks, for example: to check electromagnetic compatibility of the new component 150 with respect to image quality and/or interoperability with existing components such as sensors and processing devices; to compare new data (e.g. raw data, log files) with data from the existing configuration and to analyze parameters for calibration or optimization of dynamic driving of the devices to obtain optimal image performance; to check the new component 150 with respect to patient safety. In one such example, the new component 150 comprises a dedicated service tool that uses adapted phantom and/or sensor measurements (dB/dt, radiation dose device, etc.) for calibration to guarantee patient safety and prevent damage to existing or new components.

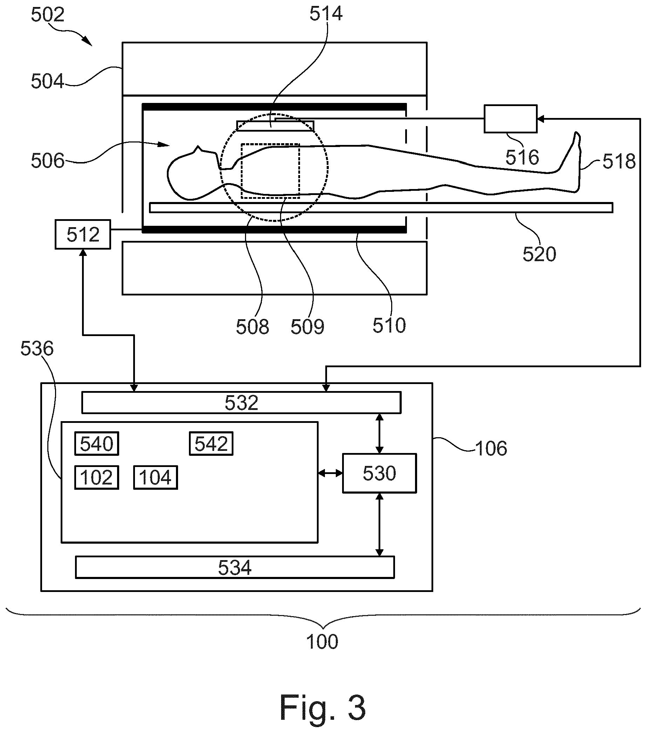

FIG. 3 shows the medical imaging system 100 in more detail, to illustrate examples of individual components which may be upgraded. In this non-limiting example, the medical imaging system 100 comprises a magnetic resonance imaging (MRI) system 502.

The medical imaging system 100 is controlled by a system controller 106, which is shown as being implemented using a processor 530 connected to a network interface 532, to a user interface 534, and to a computer memory 536.

The magnetic resonance imaging system 502 comprises a magnet 504. The magnet 504 is a superconducting cylindrical type magnet with a bore 506 through it. Within the bore 506 there is an imaging zone 508 where the magnetic field is strong and uniform enough to perform magnetic resonance imaging. A field of view 509 is shown within the imaging zone 508 within which k-space data is acquired. A subject 518 is shown as being supported by a subject support 520 such that at least a portion of the subject 518 is within the imaging zone 508 and the field of view 509. Within the bore 506 of the magnet there is also a set of magnetic field gradient coils 510 used for acquisition of k-space data within the imaging zone 508. The magnetic field gradient coils 510 are connected to a magnetic field gradient coil power supply 512. The power supplied to the magnetic field gradient coils 510 is controlled by the controller 106 as a function of time and may be ramped or pulsed. Adjacent to the imaging zone 508 is a radio-frequency coil 514 for manipulating the orientations of magnetic spins within the imaging zone 508 and for receiving radio transmissions from spins also within the imaging zone 508. The radio-frequency coil 514 is connected to a radio frequency transceiver 516 which in turn is connected to the hardware interface 532. During an image acquisition operation, the processor 530 sends pulse sequence commands which cause the magnetic resonance imaging system 502 to acquire measurement data, that is, k-space data.

Various components of the medical imaging system 100 may be upgraded via replacement by, or supplementation by, new (upgraded) components.

In one embodiment, the new component 150 is the radio-frequency coil 514 (which is shown as a body coil but may equally be a head coil), which is upgraded with a sensor module comprising additional temperature sensors to be used for enhanced monitoring of the environmental conditions, with particularly emphasis on overload or critical conditions. The additional temperature sensors enable more precise temperature monitoring, indicating inhomogeneity and also situations where environmental temperatures could lead to excessive temperatures at the coil 514, which may influence not only the performance but also the aging risk of the medical imaging system 100. The new component 150 upgrades the medical imaging system 100 to include not only adapted protocols for controlling its system functionality, but also the maintenance module 152 upgrades the maintenance functionality of the medical imaging system 100 to include checks on the usage of the new sensors, as part of a sensor guided maintenance routine, to monitor those areas where excessive temperatures have been measured.

In one another embodiment, the new component 150 replaces the gradient coils 510 (and/or their gradient amplifiers) of the MRI system 502 to enhance the system functionality. The new gradient coils need to be compatible with existing components such as the RF coil 514. All incompatible components need to be removed and replaced. The new component may use mechanical and/or electrical keying to ensure compatibility with the existing components. Additionally or alternatively, the upgrade procedure 200 as described herein may comprise in this embodiment a check to ascertain that new and old components are not inappropriately mixed, which could lead to breakdown of the system 100. The upgrade procedure may ensure compatibility by enforcing a systematic and controlled way of exchanging individual components, which may be especially useful for less experienced service personnel.

In another embodiment, the new component 150 provides an upgrade from an analogue data transmission path to a digital data transmission path. For example, the RF body coil 514 comprising analogue data transmitting cables may be upgraded to comprise a digital interface with optical fiber connections. Besides this upgrade to the system functionality, the maintenance module 152 additionally upgrades the maintenance functionality of the system 100 to include routines for the cleaning and functionality check of the connectors, to adapt the monitoring software to the new failure modes, and to measure changes in the digital data loss e.g. via increasing use of error correction codes (in the CRC check).

In yet another embodiment, the new component 150 provides an upgrade to the user interface 534, for example from a switch enabling motor-controlled movement of the subject support 520 (or, in the case of a DXR system, the positioning system for the X-ray tube and/or detector) to a force feedback and force-to-speed sensing interface which allows for more flexible and more precise positioning. Again, alongside this upgrade to the system functionality, the maintenance module 152 upgrades the maintenance functionality to include new maintenance routines as different checks become necessary. In addition, the new interface provides additional data about the usage of the system 100 and this leads to a better understanding of whether the system 100 is used in a "system friendly" way or whether the operation is carried out with critical loads and usage close to stress and force limits of the components, which is analyzed using the upgraded maintenance functionality.

In a further embodiment, the new component 150 provides an upgrade to a temperature simulation tool 540 of the medical imaging system 100. This may be more useful in the case that the medical imaging system comprises an X-ray system (e.g. CT or DXR). A more precise simulation of the temperatures based on an improved physical model enables a more precise prediction of the temperature at all components depending on the operating environment. Additional sensors may be provided to validate the simulation results and/or calibrate settings to a more precise level. The upgrade enables improved usage of the system 100, reduced waiting times for patients (as cooling down times may be reduced), and also more balanced operation with less stress on the components. This upgraded system functionality is supplemented by upgraded maintenance procedures, which include the monitoring of parameters including the temperature profiles and allowed temperature ranges. Additionally, the physical model may be upgraded to enable prediction of failure depending on temperature and operation. In any of the embodiments described herein, a certification and document module 542 may be provided to obtain the necessary documentation and certification pertaining to the upgraded system 100, especially where the system comprises a diagnostic system. This may comprise obtaining pre-existing certificates, for example when the new component is a certified component, or generating the required documents for an expedited certification process. The certification and document module 542 may receive, as input, data relating to local regulations, the new and/or old configuration, validation, and/or safety tests.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal may be included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

## Claims

1. An upgrade component configured to be integrated into a medical imaging system, wherein the upgrade component comprises:
system circuitry configured to upgrade system functionality of the medical imaging system; and
maintenance circuitry configured to upgrade maintenance functionality of the medical imaging system.

2. The upgrade component of claim 1, wherein the maintenance circuitry is configured to upgrade the maintenance functionality of the medical imaging system by providing upgraded maintenance functionality adapted to the upgraded system functionality.

3. The upgrade component of claim 1 or 2, wherein the maintenance circuitry is configured to upgrade the maintenance functionality of the medical imaging system by providing a hardware upgrade as well as a software upgrade to the maintenance functionality of the medical imaging system.

4. The upgrade component of any preceding claim, wherein the upgrade component is configured to execute, following its connection to the medical imaging system, an upgrade procedure wherein instructions are sent from the maintenance circuitry of the upgrade component to the medical imaging system.

5. The upgrade component of claim 4, wherein the instructions cause the medical imaging system to update its own maintenance circuitry with information on how to maintain the upgrade component.

6. The upgrade component of claim 4 or 5, wherein the instructions cause the medical imaging system to update a log file to record information from the upgrade component.

7. The upgrade component of any of claims 4-6, wherein the instructions indicate whether the maintenance functionality of the medical imaging system is to be adapted to the operation of the upgrade component.

8. The upgrade component of any of claims 4-7, wherein the instructions indicate whether the upgrade component will increase demands on the operation of one or more components of the medical imaging system.

9. The upgrade component of any of claims 4-8, wherein the upgrade procedure comprises the upgrade component receiving a confirmation from the medical imaging system that the instructions have been successfully actioned.

10. The upgrade component of any of claims 4-9, wherein the upgrade procedure comprises the upgrade component receiving a conflict indication from the medical imaging system, wherein the conflict indication indicates that the maintenance circuitry of the upgrade component is to adapt its operation.

11. The upgrade component of claim 10, wherein the upgrade component is configured to adapt its operation by executing one or more tasks previously executed by the medical imaging system.

12. The upgrade component of any preceding claim, wherein the system circuitry is configured to upgrade the system functionality of the medical imaging system by upgrading an analogue data transmission path to a digital data transmission path, and wherein the maintenance circuitry is configured to upgrade analog signal quality monitoring circuitry of the medical imaging system to digital signal quality monitoring circuitry.

13. A medical imaging system comprising the upgrade component of any preceding claim.

14. A method of upgrading a medical imaging system, the method comprising integrating an upgrade component into a medical imaging system, wherein the upgrade component comprises system circuitry configured to upgrade system functionality of the medical imaging system and maintenance circuitry configured to upgrade maintenance functionality of the medical imaging system.

15. A computer-readable medium comprising instructions which, when executed by a computing system, cause the computing system to execute the upgrade procedure of any of claims 4-11.
